# EUROPÄISCHE PATENTSCHRIFT

(11) **EP 1 574 836 B1**
(45) Veröffentlichungstag und Bekanntmachung des Hinweises auf die Patenterteilung: **24.10.2007**
(21) Anmeldenummer: 05005210.9
(22) Anmeldetag: 10.03.2005
(51) Int. Cl.: G01N 1/22, B01L 11/00

(54) **Probenentnahme-Verfahren und Analysen-Kit zur Durchführung einer Schadstoffanalyse in Innenräumen**
Method and test kit for testing of indoor air for harmful substances
Procédé et kit d'essai pour detection de substances nocives à l'air interieur

(30) Priorität: 12.03.2004 DE 102004012105
(43) Veröffentlichungstag der Anmeldung: 14.09.2005
(73) Patentinhaber: Führer, Gerhard, Dr., 97267 Himmelstadt (DE)
(72) Erfinder: Führer, Gerhard, Dr., 97267 Himmelstadt (DE)
(74) Vertreter: Pöhner, Wilfried Anton, Dr.

(56) Entgegenhaltungen:
- DE-A1- 4 439 433
- GB-A- 2 274 513
- NANCY K. WILSON ET AL: "Sampling Polycyclic Aromatic Hydrocarbons and Related Semivolatile Organic Compounds in Indoor Air" INDOOR AIR, Bd. 4, 1991, Seiten 513-521, XP002329445
- J. DAVID MILLER ET AL: "Air Sampling Results in Relation to Extent of Fungal Colonization of Building Materials in Some Water-Damaged Buildings" INDOOR AIR, Bd. 10, 2000, Seiten 146-151, XP002329443
- SA PETRONELLA ET AL: "CLEARING THE AIR: A MODEL FOR INVESTIGATING INDOOR AIR QUALITY IN TEXAS SCHOOLS" PROCEEDINGS: INDOOR AIR 2002, 2002, Seiten 812-817, XP002329444
- A.-L. PASANEN: "A Review: Fungal Exposure Assessement in Indoor Environments" INDOOR AIR, Bd. 11, 2001, Seiten 87-98, XP002329446
- JOHN MERRILL: "Controlling Molds and Mildew" MAINTAINING YOUR HOME, 2002, XP002329447

## Beschreibung

Die Erfindung betrifft ein Verfahren zur Probennahme für die nachfolgende Durchführung einer Analyse zum Schadstoffnachweis (Schadstoffanalyse) gemäß Oberbegriff des Anspruchs 1. Desweiteren betrifft die Erfindung einen Analysen-Kit für die Durchführung derartiger Verfahren zum Schadstoffnachweis (Schadstoffanalyse) in Innenräumen.

Mit der Verwendung moderner Baustoffe und synthetischer Bauzusatzstoffe hat das Auftreten von Schadstoffen in Innenräumen zugenommen. Schadstoff im Sinne des erfindungsgemäßen Verfahrens sind chemische Verbindungen sowie Mikroorganismen und deren Bestandteile. Bisherige Analyseverfahren sind entweder nur auf den Nachweis von nichtmikorbiellen oder nur von mikrobiellen Schadstoffen ausgerichtet.

Im Rahmen von Studien des Bundesgesundheitsamtes zwischen 1982 und 1985 wurden in Wohnhaushalten flüchtige organische Verbindungen (Volatile organic compounds, VOC) gemessen und 55 verschiedene VOCs in der Raumluft bestimmt. Identifiziert werden konnten geradkettige und verzweigte Alkane, Cycloalkane, aromatische Verbindungen, Terpene, Alkohole, Carbonyl- und verschiedene chlororganische Verbindungen. Die durch derartige Luftschadstoffe verursachten gesundheitlichen Schäden und Beeinträchtigungen können in ihrer Schwere und Verschiedenheit erheblich sein. Der qualitative und quantitative Nachweis der Schadstoffe ist der erste Schritt bei der Beurteilung des Gefahrenpotentials.

In Innenräumen können insbesondere die nachfolgend genannten Schadstoffklassen auftreten: Biozide, Flammschutzmittel, polyzyklische aromatische Kohlenwasserstoffe (PAK), Polychlorierte Biphenyle (PCB), Aldehyde, Weichmacher und flüchtigen organischen Verbindungen sowie die flüchtigen gasförmigen und partikulären Schimmelpilzbestandteile, die von mit Schimmelpilz belasteten Material abgegeben werden.

Schimmelpilzen bestehen aus einem wurzelähnlichen Geflecht (Myzel), dem Fruchtkörper und den als Verbreitungseinheit dienenden Sporen. Die Größe der Sporen liegt typischerweise in einer Größe von 2-25 Mikrometern. Während flüchtige Stoffwechselprodukte der Schimmelpilze (microbiological organic compounds, MVOC) MVOCs geruchsauffällig sind, können die ebenfalls von den Schimmelpilzen stammenden und in deren Sporen enthaltenen Mykotoxine toxisch wirken. Toxische Wirkung insbesondere auf Haut und Schleimhäute haben auch die Zerfallsprodukte, sog. Glucane, aus der Zellwand von Schimmelpilzen. Beispielsweise ist von Aflatoxinen und anderen Mykotoxinen bekannt, dass sie cancerogen sind. Die Bezeichnung "Schimmelpilze" ist eine umgangssprachliche Bezeichnung und keine wissenschaftlich systematische Einheit. Das Spektrum der allergischen Reaktionen auf Schimmelpilze reicht von Hautreizungen, grippeartigen Beschwerden über schwere Erschöpfungszustände bis hin zu Schwindel so wie Gedächtnis- und Sprachstörungen. Außerdem kann es zu verschiedensten Erkrankungen der Atemwege kommen.

Der verlässliche Nachweis von Sporen der Schimmelpilze erfolgt durch Kultivierung auf unterschiedlichen Nährböden. Zur Erfassung kultivierbarer und nicht kultivierbarer Sporen kommen außerdem mikroskopische Analysen zum Einsatz.

Die Probennahme chemischer Verbindungen in Innenräumen ist in der Richtlinie VDI-4300 (Blatt 1-8) geregelt. Hinsichtlich der Probennahme organischer Luftschadstoffe in Innenräumen hat auch das Bayerische Landesamt für Umweltschutz einen Leitfaden (BayLfU 2003- PS1) herausgegeben. In beiden Dokumenten steht allein die Analyse organischer Verbindung nicht biologischen Ursprungs im Vordergrund. Die Analyse flüchtiger organischer Verbindungen mikrobiologischen Ursprungs (z.B. Mikrobiological Volatile Organic Compounds, MVOC) oder von Mykotoxinen sowie von partikulären Schimmelpilzbestandteilen, wird darin nicht berücksichtigt.

Die Divergenz gutachterlicher Schadstoffanalysen von identischen Innenräumen zeigt, dass der Nachweise einzelner Schadstoffe oder Schadstoffklassen durch eine möglichst ganzheitliche Analyse aller nachweisbaren Schadstoffe ersetzt werden sollte. Wird bei der Analyse flüchtiger Schadstoffe auch der Nachweis der Stoffwechselprodukte von Schimmelpilzen angestrebt, so fehlen bezüglich der Reihenfolge bei der Probennahme Vorgaben, die ein standardisiertes Vorgehen sicherstellen.

Aus Dokument DE 4 439 433 A1 ist ein Verfahren zur quantitativen Analyse eines definierten Luftinhaltsstoffs auf immunologischer Basis bekannt. Zur Durchführung des Verfahrens wird eine Vorrichtung verwendet, die sich aus einem ersten und aus einem zweiten Probennehmerteil zusammensetzt. Das Kernstück des ersten Probennehmerteils ist ein Filter der partikelgebundene Luftinhaltsstoffe (Aerosole) aufnimmt. Das zweite Probennehmerteil ist ebenfalls ein Filter oder ein Absorbtionsmittel, insbesondere ein Silicagel. Beide Filter bestehen aus einem Material, das gegenüber dem Analyten inert ist. Zur Probennahme wird eine definierte Luftmenge durch die Filteranordnung gesogen. Nach der Probennahme werden der erste und der zweite Filter gemeinsam einem Lösungsmittelbad zugeführt, indem sowohl die partikelgebundenen als auch die sorbtiv gebundenen Analyten aus den Filtern in Lösung gebracht werden. Der Gehalt des Lösungsmittelbades an Luftinhaltsstoffen wird unter Ausführung eines analytspezifischen Immuno-Assays quantitativ bestimmt.

Weiterhin ist aus Dokument DE 3 811 245 A1 ein Verfahren zur Ermittlung der Schadstoffbelastung der Außenluft und der Luft in Innenräumen mit Hilfe von Bioindikatoren bekannt. Als Bioindikatoren wird der Einsatz von Kulturen von Pilzen vorgeschlagen. Die Pilze dienen als Frühwarnsystem zur Indikation einer Gesamtschadstoffbelastung der Luft. Zur Beurteilung der vorliegenden Schadwirkung werden Veränderungen qualitativer und quantitativer Merkmale der Bioorganismen nach Exposition unter standartisierten Bedingungen mit den entsprechenden Merkmalen nicht exponierter Textorganismen (Kontrollproben) verglichen. Bei der Auswertung stehen morphologische Kriterien im Vordergrund. Die Verwendung solcher Bioindikatoren erlaubt keinen exakten quantitativen Nachweis der einzelnen Schadstoffe, obwohl zur weiteren Analyse der Pilze nach Schadstoffexposition der Einsatz biochemischer und molekularbiologischer Verfahren vorgesehen ist. Außerdem erfordert die Auswertung der morphologisch veränderten Bioindikatoren, die nicht nur im Ablesen eines Meßwertes besteht, nachteiligerweise die Erfahrung eines Experten.

Aus der GB 2 274 513 ist eine Überwachungseinheit für Luft bekannt, in welcher die Luftprobe aufgeteilt und jeweils unterschiedliche Untersuchungen durchführenden Analyseeinheiten zugeführt wird. Eine Untersuchung des Vorkommens von polyzyklischen aromatischen Kohlenwasserstoffen (PAK) in der Innenluft unter verschiedenen Bedingungen werden in der Untersuchung von Wilson et al. "Sampling polyzyclic aromatic hydrocarbons and related semivolatile organic compounds in Indoor air", veröffentlicht in der Zeitschrift Indoor Air; 4, 513 ff veröffentlicht. Untersucht wird einzig die Konzentration unter verschiedenen Einflüssen. In der gleichen Zeitschrift wurde eine Untersuchung des Vorkommens von Pilzkolonien in der Gebäudeluft berichtet (Indoor Air 2000; 10: 146 - 151. Verfasser: Miller et al.). In einer weiteren, in der selben Zeitschrift veröffentlichten Untersuchung wird die Luftqualität an Schulen untersucht (Indoor Air; 2002: 812 - 817 Verfasser Petronella et al.). Hierbei werden die Werte von Formaldehyd, flüchtigen organischen Verbindungen (VOC's), Ozon berücksichtigt, wobei die Messung mit Gas- oder Flüssigkeitschromatographen unter Laborbedingungen durchgeführt werden.

Aufgabe des erfindungsgemäßen Verfahrens vor diesem Hintergrund ist die Optimierung der Probennahme bei der Schadstoffanalyse von Innenräumen (Innenraum-Check) mit dem Ziel eines möglichst umfassenden genauen qualitativen und quantitativen Nachweises in Kombination mit der Erfassung gasförmiger Stoffwechselprodukte von Mikroorganismen und anderer luftgetragener Bestandteile mikrobiologischen Ursprungs (z. B. Mykotokine und MVOCs) bei gleichzeitiger Kostenminimierung.

Durch das erfindungsgemäße Verfahren ist neben dem qualitativen und quantitativen Nachweis von luftgetragenen Bestandteilen mikrobiologischen Ursprungs (z. B. Mykotokine oder gasförmige Stoffwechselprodukte wie MOVCs) sowie deren partikulärer Bestandteile der qualitative und quantitative Nachweis aller in die oben genannten Schadstoffklassen gehörender Einzelverbindungen sowie der durch Thumulla at al. "Schadstoff in Innenräumen-eine aktuelle Übersicht (Umwelt-Medizin-Gesellschaft, 14, 4/2001, Seite 291 bis 300) und im Leitfaden des Bayrischen Landesamtes für Umweltschutz "Organische Luftschadstoffe in Innenräumen - Probennahme, Messung und Bewertung" (BayLfU 2003- PS1)sowie in der VDI Richtlinie 4300 genannten Einzelverbindungen vorgesehen.

Zur Lösung der Aufgabe schlägt die Erfindung ein Verfahren vor, das durch die Merkmale des Kennzeichens von Anspruch 1 beschirieben ist.

Der Kerngedanke des erfindungsgemäßen Verfahrens besteht in der Kombination verschiedener Analyseverfahren zum Nachweis von Schadstoffen mikrobiellen Ursprungs, wie beispielsweise MVOCs und Mykotoxinen, neben VOCs, sowie von Partikeln wie Sporen mikrobiellen Ursprungs und gasförmigen bzw. staubgebundenen chemischen Verbindungen durch Emission aus (Bau-)Materialien. Der Nachweis der Schadstoffe im Rahmen des erfindungsgemäßen Verfahrens erfolgt unter besonderer Beachtung der Störanfälligkeit von Raumluftmessungen, insbesondere der der MVOC-Messung. Bereits ein Pilzbefall der Haut oder Hautflora des Experimentators kann im Extremfall die Messung, die dem Nachweis vergleichsweise geringer Konzentrationen (ng/m³) dient, stören.

Das erfindungsgemäße Verfahren zur Probennahme wird dadurch charakterisiert, dass aufbauend auf der Probennahme sowohl die in der Luft enthaltenen Schadstoffe, als auch auf sichtbare und versteckte (nicht sichtbare) Schimmelpilzschäden zurückgehenden flüchtigen Verbindungen (MVOC) analysierbar sind. Dabei ist zu bedenken, dass eine umfassende Schimmelpilzanalyse zum einen den Nachweis der mikrobiologischen flüchtigen organischen Verbindungen (MVOC) und zum anderen die Analyse der partikulären Schimmelpilzbestandteile, wie z. B. die Schimmelpilzsporen, mit mikrobiologischen Verfahren umfasst. Obwohl eine isolierte Analyse der MVOCs oder eine isolierte Analyse des sichtbaren Schimmelpilzbefalls bereits wesentliche Aussagen zur Schadstoffbelastung ermöglicht, ist zu bedenken, dass nur die zusammenschauende Auswertung der verschiedenen hier vorgesehenenen Analyseverfahren eine zuverlässige Aussage über die Schadstoffbelastung von Innenräumen erlaubt. Das für den Schimmelpilzbefall und die Schimmelpilzanalyse gilt analog für Bakterien.

Die Materialproben werden durch die dem Fachmann bekannten Möglichkeiten von den verschiedenen Baumaterialien genommen.

Die Proben der Raumluft werden vorzugsweise im Hauptaufenthaltsraum der Wohnung genommen. Aufgrund der bei Gasen schnell erfolgenden Diffusion kann bei Räumen innerhalb eines Stockwerks davon ausgegangen werden, dass die Schadstoffkonzentrationen vergleichbar sind. Im Gegensatz zu den Raumluftproben sollten Staubproben, die u.a. dem Nachweis schwerflüchtiger organischer Verbindungen (SVOC) dienen, dagegen aus allen Hauptaufenthaltsräumen entnommen werden, da die staubgebundenen Verbindungen (SVOC) bzw. die partikulären organischen Verbindungen (particulate organic matter, POM) und/oder (Schwer-)Metallen weniger flüchtig sind.

Die anderen flüchtigen Luftschadstoffe umfassen die leicht flüchtigen organischen Verbindungen (WOC) und die flüchtigen organischen Verbindungen (VOC). Im Rahmen des Verfahrens ist vorgesehen, dass die Probennahme dieser beiden Schadstoffklassen vor Entnahme der Staubproben erfolgt. Staubproben sind erfahrungsgemäß eher inhomogen, sodass leichte Veränderungen der Bedingungen zu Probennahme bei den Staubproben die geringste Auswirkung auf die Meßgenauigkeit haben.

Zur Erreichung einer möglichst hohen Meßgenauigkeit werden im Rahmen des erfindungsgemäßen Verfahrens bei der Entnahme der anderen organischen Luftschadstoffe zuerst die Proben zum Nachweis der leicht flüchtigen organischen Verbindungen (WOCs) und der flüchtigen organischen Verbindungen (VOCs) entnommen. Dadurch wird berücksichtigt, dass die Anwesenheit des Experimentators auf die Konzentrationen der schnell diffundierenden WOCs für möglichst kurze Zeit einen störenden Einfluss haben kann. Etwa in einem Zeitraum von 12 Stunden vor der Raumluftprobennahme sollte sich keine Person in dem Raum aufgehalten haben. Die Anwesenheit von Personen kann zur Artefakt-Bildung bei der MVOC-Probennahme führen. Zur Standardisierung sollte, entsprechend den VDI-Richtlinien 4300, 8 Stunden vor der Probennahme nicht gelüftet worden sein.

Im Rahmen des erfindungsgemäßen Probennahme-Verfahrens ist der Einsatz verschiedenster Adsorptionsmittel zur Entnahme der flüchtigen organischen Schadstoffe vorgesehen. In Betracht kommen insbesondere Aktivkohle, Dinitrophenylhydrazin, Polyurethan oder Silicagel. Derartige Adsorber sind dem Fachmann auch unter den Handelsnamen TENAX (2,6 Diphenylparaphenylenoxid) und Florisil (Magnesium-Silcagel) oder XAD-Adsorber bekannt. Es ist vorgesehen, dass die Adsorptionsmaterialen gegenüber den Analyten chemisch inert sind oder diese, wie beispielsweise Dinitrophenylhydrazin in einen chemisch stabilen Zustand überführen.

Zur Probennahme aller flüchtigen organischen Schadstoffe ist vorgesehen, dass mit Hilfe einer Pumpe ein jeweils definiertes Volumen der Raumluft durch eine mit einem Adsorptionsmittel gefülltes Adsorberröhrchen gepumpt wird.

Zum Nachweis der mikrobiologischen flüchtigen organischen Verbindungen (MVOCs), die normalerweise in sehr geringen Konzentrationen (ng/m³) nachgewiesen werden müssen, wird ein Volumen von 2-3 Liter bei einer Durchflussrate von 0,1 - 0,2 Umin durch ein TENAX-Adsorber gepumpt. Bei alternativer Verwendung eines mit Aktivkohle befüllten Adsorberröhrchens zur Probennahme der MVOCs wird ein Luftvolumen von mehr als 10 Litern benötigt.

Die VOC-Probennahme erfolgt bei mit Aktivkohle befüllten Adsorberröhrchen von einem Luftvolumen von etwa 100 Litern bei einem Durchfluss von ca. 1 - 2 Umin. VOCs können alternativ auch mit TENAX-, FLORISIL-Adsorbern oder anderen Adsorptionsmitteln durchgeführt werden.

Als Adsorptionsmittel für Aldehyde wird Dinitrophenylhydrazin (DNPH) eingesetzt. Dabei werden 50 Liter bei einer Flussrate von etwa 1,5 L/min, durch das Adsorptionsmittel gepumpt. In der DNPH-Matrix werden die reaktiven Aldehyde im Rahmen der Probennahme in stabile Verbindungen überführt werden, deren Nachweis die genaue Bestimmung der zum Zeitpunkt der Probennahme vorliegenden Aldehyd-Konzentration erlaubt. Aldehyde-Proben können alternativ auch mit Hilfe von XAD-2-Röhrchen genommen werden.

Die Nachweisgrenzen der einzelnen Verbindungsklassen sind für MVOC (ca. 10 ng/m³), VOC (ca. 1 µg/m³), SVOC/Staub (ca. 0.1 mg/kg) und Aldehyden (ca. 1-5 µg/m³).

Für die Analyse der nachdem erfindungsgemäßen Verfahren entnommenen Proben ist der kombinierte Einsatz chemischer, biochemischer und physikalischer Methoden vorgesehen. Die zum Nachweis flüchtiger Verbindungen vorgesehener Verfahren umfassen gaschromatographische und flüssigchromatographische Verfahren, sowie die UV-Spektroskopie und die Massenspektroskopie. Die Analyse der (Schwer-)Metalle erfolgt durch Atomemissionsspektroskopie (AES), insbesondere durch Inductive-Coupled-Plasma-AES.

Zur Analyse der flüchtigen Verbindungen, insbesondere der von den Schimmelpilzen stammenden MVOCs sowie bei der Analyse der in partikulärer Form vorliegenden Schimmelpilzbestandteile kommen biochemische Analyseverfahren zum Einsatz die sowohl mikrobiologische, molekularbiologische als auch immunologische Verfahren einschließen.

Zur Probennahme der staubgebundenen Schadstoffe (SVOC) und partikulären organischen Verbindungen (particulate organic matter, POM) oder (Schwer-)Metallen wird ein mit einem frischen Saugbeutel ausgeschatteter Staubsauger verwendet. Der vorzugsweise 7 Tage alte Staub (Frischstaub) wird durch normales" Staubsaugen des Raumes gesammelt, wobei die SVOC-Analyse ca. 1 Gramm (bei POM und beim (Schwer-)Metall-Nachweis deutlich mehr) Staub erfordert. Bei Verwendung eines Staubsaugeraufsatzes zur fraktionierten Aufnahme der Staubpartikel mit einer Größe von 200 bis 63 Mikrometer ist deutlich weniger als 1 Gramm Staub erforderlich.

Die Erfindung betrifft des weiteren einen Analysen-Kit mit Adsorptionsmitteln und Trägermaterialien zur Probennahme für die nachfolgende Durchführung einer Analyse zum Schadstoffnachweis (Schadstoffanalyse) in Innenräumen.

Eine Zusammenstellung von Adsorptions- und Trägermaterialien als Kit zur Durchführung einer aktiven Probennahme z.B. mittels Pumpen mit nachfolgender Schadstoffanalyse ist nicht bekannt. Lediglich zum Nachweis von Aldehyden ist in Apotheken ein Schnelltest erhältlich, der genauso wie der Nachweis von Pentachlorphenol (PCP) mit Hilfe eines Pflasters vom Laien anwendbar ist. Dabei handelt es sich jedoch jeweils nur um den halbquantitativen Einzelnachweis von Substanzen, der keine umfassende Aussage über den Schadstoffgehalt der Luft erlaubt und deshalb eine umfassende Analyse nicht ersetzt.

Mit Hilfe des erfindungsgemäßen Schadstoffanalysen-Kits soll die einheitliche Durchführung der Probennahme im Rahmen einer Innenraum-Schadstoffanalyse sichergestellt und vereinfacht werden.

Der Kit enthält bei Bedarf neben den mit einem oder verschiedenen Adsorptionsmitteln befüllten Adsorberröhrchen Trägermaterial zur Aufnahme von staubgebundenen Schadstoffen, Schadstoffpartikeln und Schimmelpilzbestandteilen technische Geräte zur Bewältigung der Probennahme wie Pumpen und Gasuhren sowie Verpackungsmaterial zum fachgerechten Transport der Proben. Die Anschlüsse der Probennahmegeräte sind mit denen der Adsorberröhrchen kompatibel.

Die Adsorptionsmittel und Trägermaterialien zeichnen sich dadurch aus, dass sie gegenüber den Analyten inert sind oder diesen, wie beispielsweise Dinitrophenolhydrazin (DNPH) in einen chemischen stabilen Zustand überführen. Die Überführung in einen reaktionsstabilen Zustand ist insbesondere zum verlässlichen Nachweis von Aldehyd-Verbindungen erforderlich.

Als in Adsorberröhrchen eingebrachte Adsorptionsmittel kommen Aktivkohle, Dinitrophenolhydrazin, Silicagel, Polyurethan und ähnliche Adsorptionsmittel in Frage, wobei Nitrophenolhydrazin insbesondere zur Probennahme der Aldehydverbindungen gewählt wird.

Es versteht sich von selbst, dass die zur Probennahme eingesetzten Adsorberröhrchen mit den handelsüblichen Probenahmegeräten und Analysegeräten kompatibel sind.

Als Trägermaterial zur Entnahme von Staubproben sind Staubsaugerbeutel, die im Zusammenhang mit einem Staubsauger eingesetzt werden sowie Staubwischtücher vorgesehen. Letztere können z.B. bei flächigem Schimmelpilzbefall der Wände zum Einsatz kommen. Nach entsprechender Aufarbeitung, z. B. durch Herauslösen der Schimmelpilzbestandteile aus dem Gewebe, sind die Proben einer biochemischen Analyse zugänglich. Die biochemische Analyse kann dabei molekularbiologische, mikrobiologische sowie immunologische Methoden umfassen.

Ziel des erfindungsgemäßen Kits ist die Standartisierung der Probennahme. Dazu gehört auch die einheitliche Behandlung der Proben beim Versenden. Zu diesem Zweck liegen dem Analysen-Kit Gefäße und Schutzhüllen sowie Umschläge zur fachgerechten Versendung der Proben und Adsorptionsmittel bei.

### Definitionen /Abkürzungen

- WOC: leichtflüchtige organische Verbindungen (very volatile organic compounds) mit einem unteren Siedepunktsbereich von < 0° bis zu einem oberen Siedepunktsbereich von Microbiological volatile organic compounds 50 -100° C. Beispiele für WOC sind Aceton, C₂-C₅ Alkane sowie Formaldehyd und Acetaldehyd. Beispiele für VOC sind C₆-C₁₅ Alkane, Benzol und dessen Derivate, Ester, längerkettige Alkohole, Ketone ab C₄, Terpene, Siloxane, EEMA, höhere Aldehyde.
- VOC: Flüchtige organische Verbindungen (volatile organic compounds) mit einem unteren Siedepunktsbereich von 50 - 100° C und einem oberen Siedepunkt von 240 - 260 °C.
- SVOC: Schwerflüchtige organische Verbindungen (semivolatile organic compounds) mit einem unteren Siedepunkt von 240 - 260 und einem oberen Siedepunkt von 380 - 400°C. Beispiele für SVOC sind Holzschutzmittel, Weichmacher wie sie insbesondere bei PVC-Produkten eingesetzt werden, leichtflüchtige PAK, Flammenschutzmittel sowie PCB.
- POM: Partikuläre organische Verbindungen oder partikelgebundene organische Verbindungen (particulate organic matter) mit einem Siedepunkt von > 380° C. Beispiele für POM sind Pyrethroide, Sulfonamide und schwerflüchtige PAK. Sie sind aufgrund ihres hohen Siedepunktes in der Raumluft nicht nachweisbar, können aber durch die Probennahme von Staub nachgewiesen werden.
Die oben stehenden Definitionen entsprechen der "Klassifizierung von organischen Verbindungen im Innenraum" der World Health Organisation (WHO) vom 1989.
- MVOC: Mikrobiologische flüchtige organische Verbindungen (microbiological volatile organic compounds) sind die auf Schimmelpilze zurückgehenden Stoffwechselprodukte, die in der Raumluft nachgewiesen werden können.
- PAK: Polycyclische aromatische Kohlenwasserstoffe
- PCP: Pentachlorphenol
- PCB: Polychlorierte Biphenyle

## Patentansprüche

1. Verfahren zur Probennahme für die nachfolgende Durchführung einer Analyse zum Nachweis von Schadstoffen, die sowohl in der Luft, als auch stab- oder materialgebunden vorliegen, separat erfasst und analysiert werden, wobei
- die Analyse in Innenräumen stattfindet,
- bei der Probennahme
- zuerst die Raumluftproben zum Nachweis der gasförmigen Stoffwechselprodukte von Mikroorganismen und gegebenenfalls anderer luftgetragener Bestandteile mikrobiologischen Ursprungs, und
- nachfolgend die Proben zum Nachweis anderer flüchtiger organischer Luftschadstoffe in der Raumluft, zu denen leicht flüchtige organische Verbindungen, die durch einem unteren Siedepunktsbereich von < 0° bis zu einem oberen Siedepunktsbereich von 50 - 100° C definiert sind, und in der Raumluft vorliegende schwerflüchtige Verbindungen, die durch einen unteren Siedepunkt von 240 - 260° C und einem oberen Siedepunkt von 380 - 400°C definiert sind, und flüchtige organische Verbindungen, die durch einen unteren Siedepunktsbereich von 50 - 100° C und einem oberen Siedepunkt von 240 - 260 °C definiert sind, zählen, entnommen und/oder
- nachfolgend die Staubproben zum Nachweis schwerflüchtiger organischer Verbindungen, die durch einen unteren Siedepunkt von 240 - 260° C und einem oberen Siedepunkt von 380 - 400°C definiert sind, partikulärer organischer Verbindungen, die durch einen Siedepunkt über 380°C definiert sind, und/oder von (Schwer-) Metalle genommen werden, und
- die Proben chemischen und/oder physikalischen und/oder biochemischen Analysenverfahren zugeführt werden.

2. Verfahren nach Anspruch 1, **dadurch gekennzeichnet, dass** auf der Grundlage der Probennahme sowohl die Analyse chemische Emissionen aus (Bau-)Materialien, als auch eine Schimmelpilzanalyse durchgeführt wird, wobei die Schimmelpilzanalyse die Analyse der gasförmigen Stoffwechselprodukte von Mikroorganismen und/oder die Analyse der partikulärer Schimmelpilzbestandteile umfassen kann.

3. Verfahren nach einem der vorhergehenden Ansprüche, **dadurch gekennzeichnet, dass** bei der Entnahme der anderen organischen Luftschadstoffe zuerst die Proben zum Nachweis der leichtflüchtigen organischen Verbindungen und nachfolgend die der flüchtigen organischen Verbindungen sowie die in der Raumluft vorliegenden schwerflüchtigen organischen Verbindungen genommen werden.

4. Verfahren nach einem der vorhergehenden Ansprüche, **dadurch gekennzeichnet, dass** die Probennahme flüchtiger organischer Schadstoffe aus der Raumluft durch das Hindurchleiten eines definierten Volumens der Raumluft durch ein mit einem Adsorptionsmittel gefüllten Adsorberröhrchen erfolgt.

5. Verfahren nach einem der vorhergehenden Ansprüche, **dadurch gekennzeichnet, dass** als Adsorptionsmittel/Matrix zur Entnahme flüchtiger organischer Schadstoffe aus der Raumluft Aktivkohle, Dinitrophenylhydrazin, Polyurethane, Zeolithe, Silicagel oder andere Adsorptionsmittel eingesetzt werden.

6. Verfahren nach einem der vorhergehenden Ansprüche, **dadurch gekennzeichnet, dass** als Adsorptionsmittel zur Entnahme gasförmiger Aldehyd-Verbindungen Dinitrophenylhydrazin und XAD-Adsorberröhrechen eingesetzt werden.

7. Verfahren nach einem der vorhergehenden Ansprüche, **dadurch gekennzeichnet, dass** die der Analyse flüchtiger Verbindungen mikrobiellen Ursprungs dienenden chemischen Analysenverfahren gaschromatographische und/oder flüssigchromatographische und/oder UV-spektroskopische und/oder massenspektroskopische Verfahren einschließen.

8. Verfahren nach einem der vorhergehenden Ansprüche, **dadurch gekennzeichnet, dass** die der Analyse der (Schwer-) Metalle die Atomemissionsspektroskopie einschließt.

9. Verfahren nach einem der vorhergehenden Ansprüche, **dadurch gekennzeichnet, dass** die der Analyse flüchtiger Verbindungen mikrobiellen Ursprungs dienenden und/oder der Analyse partikulärer Schimmelpilzbestandteile dienenden biochemischen Analyseverfahren mikrobiologische und/oder molekularbiologische und/oder immunbiologische Verfahren einschließen.

10. Verfahren nach einem der vorhergehenden Ansprüche, **dadurch gekennzeichnet, dass** staubgebundene Schadstoffe und/oder Schadstoffpartikel mit einem Staubsauger aufgenommen werden, wobei in den Staubsauger ein frischer Saugbeutel eingelegt ist und/oder der Staub durch einen Aufsatz mit definierter Korngröße gesammelt und nachfolgend einer chemischen und/oder biochemischen Analyse zugeführt wird.

11. Verfahren nach einem der vorhergehenden Ansprüche, **dadurch gekennzeichnet, dass** staubgebundene und/oder in partikulärer Form vorliegender Bestandteile von Schimmelpilzen mit einem gewebe-, papier- oder filmartigen Trägermaterial, aufgenommen und einer mikrobiologischen und/oder molekularbiologischen und/oder immunbiologischen Analyse zugeführt werden.

12. Verfahren nach einem der vorhergehenden Ansprüche, **dadurch gekennzeichnet, dass** der bei der Probeentnahme aufgenommene Staub einen bis viele, vorzugsweise sieben, Tage alt ist und dem Nachweis von schwerflüchtigen organische Verbindungen und/oder partikulären organischen Verbindungen und/oder (Schwer-)Metallen und/oder dem Schimmelpilznachweis dient.

13. Analysen-Kit zur Durchführung des Verfahrens nach einem der Ansprüche 1 -12 mit Adsorptionsmaterialien und Trägermaterialien zur Probennahme für die nachfolgende Durchführung einer Analyse zum Schadstoffnachweis (Schadstoffanalyse) in Innenräumen, wobei
- die Probennahme sowohl der Analyse der Luft auf flüchtige Luftschadstoffe als auch der Analyse auf Schimmelpilzbefall dient, und
- der Analysen-Kit
eines oder verschiedene, in Adsorberröhrchen gebundene, Adsorptionsmittel zur Sorption gasförmiger Substanzen, sowie zum Nachweis schwerflüchtiger organischer Verbindungen, partikulärer organischer Verbindungen und/oder von (schwer-) Metalle enthält.

14. Schadstoffanalysen-Kit nach Anspruch 13, **dadurch gekennzeichnet, dass** der Kit Probennahmegeräte, insbesondere eine Pumpe mit Anschlüssen, die kompatibel sind zu den Anschlüssen der Adsorberröhrchen, enthalten kann.

15. Schadstoffanalysen-Kit nach einem der Ansprüche 13 und 14, **dadurch gekennzeichnet, dass** das Trägermaterial zur Ausnahme von staubgebundenen Schadstoffen und partikulären Schadstoffen Staubsaugerbeutel oder spezielle Probennahmeköpfe zum Aufsatz auf den Staubsauger mit variabler Korngröße und/oder Wischtücher sind.

16. Schadstoffanalysen-Kit nach einem der Ansprüche 13 bis 15, **dadurch gekennzeichnet, dass** die Adsorptionsmittel und die Trägermaterialien gegenüber den Analyten inert sind.

17. Schadstoffanalysen-Kit nach einem der Ansprüche 13 bis 16, **dadurch gekennzeichnet, dass** mindestens eines der Adsorptionsmittel einen oder mehrere der Analyten in einen chemisch stabilen Zustand überführen.

18. Schadstoffanalysen-Kit nach einem der Ansprüche 13 bis 17, **dadurch gekennzeichnet, dass** die Adsorberröhrchen mit
- Aktivkohle,
- Nitrophenolhydrazin,
- Zeolithen,
- Silicagel,
- Polyurethanen oder
einem ähnlichen Adsorptionsmittel befüllt sind.

19. Schadstoffanalysen-Kit nach einem der Ansprüche 13 bis 18, **dadurch gekennzeichnet, dass** die Adsorberröhrchen mit handelsüblichen Probennahmegeräten und Analysengeräten kompatibel sind.

20. Schadstoffanalysen-Kit nach einem der Ansprüche 13 bis 20, **dadurch gekennzeichnet, dass** der Analysen-Kit auch Gefäße und/oder Schutzhüllen und/oder Umschläge zur fachgerechten Versendung der Proben und Adsorptionsmittel enthält.

21. Verwendung des nach einem der Ansprüche 13 bis 20 beschriebenen Schadstoffanalysen-Kit's zur Schadstoffanalyse in Innenräumen gemäß dem in einem der Ansprüche 1 bis 13 beschriebenen Verfahren.

## Claims

1. Sampling method for subsequently performing an analysis for detection of pollutants, which are present in the air as well as dust- or material-bound, and are separately gathered and analyzed, wherein
- analysis takes place in indoor room,
- for the sampling
- first the room-air samples are taken for detecting the gaseous metabolic products of microorganisms and, if appropriate, other airborne components of microbiological origin and
- subsequently the samples are taken for detection of other volatile organic air pollutants, which include very volatile organic compounds, which are defined by a lower boiling point range from < 0°C to an upper boiling point range of 50-100 °C, and semi-volatile compounds present in the room air, which are defined by a boiling point from 240 to 260 °C and an upper boiling point of 380 to 400 °C, and volatile organic compounds, which are defined by a lower boiling point range from 50 to 100 °C, and an upper boiling point of 240 to 260 °C, and/or
- subsequently the dust samples for the detection of semi-volatile organic compounds, which are defined by a boiling point from 240 to 260 °C and an upper boiling point of 380 to 400 °C, particulate organic compounds, which are defined by a boiling point over 380 °C, and/or of (heavy) metals are taken, and
- the samples are submitted for chemical and/or physical and/or biochemical analysis methods.

2. Method according to claim 1, **characterised in that,** on the basis of the sampling, the analysis of chemical emissions from (construction) materials, as well as mould analysis are carried out, wherein the mould analysis may comprise the analysis of gaseous metabolic products from microorganisms and/or the analysis of particulate mould components.

3. Method according to one of the preceding claims, **characterised in that,** on taking the other organic air pollutants, first the samples for detection of the volatile organic compounds are taken and then the volatile organic compounds as well as the semi-volatile organic compounds present in the room air.

4. Method according to one of the preceding claims, **characterised in that** the sampling of volatile organic pollutants from the room air takes place by passing a defined volume of the room air through an adsorber tube filled with an adsorbent.

5. Method according to one of the preceding claims, **characterised in that** the adsorbent /matrix for taking volatile organic pollutants from the room air comprise active charcoal, dinitrophenyhydrazine, polyurethanes, zeolites, silica gel, or other adsorbents.

6. Method according to one of the preceding claims, **characterised in that** the adsorbent for taking gaseous aldhehyde compounds comprise dinitrophenylhydrazine and XAD adsorber tubes..

7. Method according to one of the preceding claims, **characterised in that** the chemical analysis methods which serve for the analysis of volatile compounds of microbial origin include gas chromatographic and / or liquid chromatographic and/or UV spectroscopic and/or mass spectroscopic methods.

8. Method according to one of the preceding claims, **characterised in that** the method for the analysis of the (heavy) metals includes atomic emission spectroscopy.

9. Method according to one of the preceding claims, **characterised in that** the biochemical analysis methods which serve for the analysis of volatile compounds of microbial origin and/or for the analysis of particulate mould components include microbiological and/or molecular biological and/or immunobiological methods.

10. Method according to one of the preceding claims, **characterised in that** dust-bound pollutants and/or pollutant particles are picked up with a vacuum cleaner, a fresh vacuum cleaner bag being inserted in the vacuum cleaner and/or the dust being collected by means of an attachment with defined particle size, and then undergo a chemical and/or biochemical analysis.

11. Method according to one of the preceding claims, **characterised in that** dust-bound and/or mould components in particulate form are picked up by means of a fabric, paper or film-like carrier material, and then undergo for microbiological and/or molecular biological and/or immunobiological analysis.

12. Method according to one of the preceding claims, **characterised in that** the dust picked up during sampling is one to several, preferably seven, days old, and serves for the detection of semi-volatile organic compounds and/or particulate organic compounds and/or (heavy) metals and/or the detection of moulds.

13. Analysis kit for performing the method according to one of claims 1-12, with adsorption materials and carrier materials for sampling for the subsequent performance of an analysis for pollutant detection (pollutant analysis) in indoor rooms, wherein
- the sampling and analysis of the air for volatile air pollutants also serves for the analysis for mould attack
- the analysis kit contains one or more adsorbents, which are bound in adsorber tubes, for the sorption of gaseous substances and for the detection of semi-volatile organic compounds particulate organic compounds and/or (heavy) metals.

14. Pollution analysis kit according to claim 13, **characterised in that** the kit can contain sampling units, in particular a pump with connections, which are compatible with the connections of the adsorber tubes.

15. Pollution analysis kit according to one of claims 13 and 14, **characterised in that** the carrier material for pick-up of dust-bound pollutants and particulate pollutants are vacuum-cleaner bags or special sampling heads for attachment to the vacuum cleaner, with variable grain size and/or wiper cloths.

16. Pollution analysis kit according to one of claims 13 to 15, **characterised in that** the adsorbents and the carrier materials are inert with respect to the analytes.

17. Pollution kit according to one of claims 13 to 16, **characterised in that** at least one of the adsorbents converts one or more of the analytes into a chemically stable state.

18. Pollution analysis kit according to one of claims 13 to 17,
**characterised in that** the adsorber tubes are filled with
- activated charcoal
- nitrophenylhydrazine
- zeolites
- silica gel
- polyurethanes, or
a similar adsorbent.

19. Pollution analysis kit according to one of claims 13 to 18, **characterised in that** the adsorber tubes are compatible with commercially available sampling units and analysis equipment.

20. Pollution analysis kit according to one of claims 13 to 20,
**characterised in that** the analysis kit also contains vessels and/or protective sleeves and/or envelopes for correct dispatch of the samples and adsorbents

21. Use of the pollution analysis kit described in one of claims 13 to 20, for pollution analysis in indoor rooms according to the method described in one of claims 1 to 13.

## Revendications

1. Procédé pour le prélèvement d'échantillons pour l'exécution consécutive d'une analyse destinée à prouver la présence d'agents polluants présents tant dans l'air que sous forme liée à la poussière ou aux matériaux, enregistrés et analysés séparément, sachant que
• cette analyse a lieu dans des espaces intérieurs;
• que sont ajoutés, lors du prélèvement d'échantillons,
■ d'abord les échantillons d'air ambiant prélevés pour prouver la présence de produits du métabolisme de microorganisme et le cas échéant d'autres composants d'origine biologique portés par l'air, et
■ ensuite les échantillons prélevés pour prouver la présence d'autres agents polluants volatils de l'air dans l'air ambiant, parmi lesquels on compte les liaisons organiques très volatiles, définies comme étant celles ayant une zone inférieure d'ébullition <0°C et une zone supérieure d'ébullition, dé 50 - 100°C, et les liaisons peu volatiles présentes dans l'air ambiant, définies comme étant celles ayant une zone inférieure d'ébullition de 50-100°C et une zone supérieure d'ébullition de 240- 260°C et/ou
■ ensuite les échantillons de poussière prélevés pour prouver les liaisons organiques peu volatiles définies comme étant cettes ayant une zone inférieure d'ébullition de 240 - 260°C et une zone supérieure d'ébullition de 380 - 400°C, les liaisons organiques sous forme de particules, définies comme étant celles ayant un point d'ébullition supérieur à 380°C, et/ou les métaux (lourds), et
■ les échantillons prélevés pour les procédés d'analyse chimique, et/du physique et/ou biochimique.

2. Procédé selon la revendication 1, **caractérisé par le fait qu'**est effectuée, sur la base du prélèvement d'échantillon, tant l'analyse des émissions chimiques provenant de matériaux (de construction), qu'une analyse des moisissures, sachant que l'analyse des moisissures peut englober l'analyse des produits du métabolisme de microorganismes sous forme de gaz et/ou l'analyse des composants de moisissure sous la forme de particules.

3. Procédé selon une des revendications précédentes, **caractérisé par le fait que** sont prélevés, lors du prélèvement des autres agents organiques polluants de l'air, d'abord les échantillons destinés à prouver les liaisons organiques très volatiles et ensuite les liaisons organiques peu volatiles présentes dans l'air.

4. Procédé selon une des revendications précédentes, **caractérisé par le fait que** le prélèvement d'agents organiques polluants volatils est effectué en faisant passer un volume défini de l'air ambiant à travers un petit tube adsorbeur rempli d'un agent d'adsorption.

5. Procédé selon une des revendications précédentes, **caractérisé par le fait qu'**on emploie, en tant qu'agent d'adsorption/matrice pour prélever les agents polluants volatils de l'air ambiant, du charbon actif, du dinitrophénylhydrazine, du polyuréthane, du zéolithe, du gel de silice ou d'autres agents d'adsorption.

6. Procédé selon une des revendications précédentes, **caractérisé par le fait qu'**on emploie, en tant qu'agent d'adsorption pour prélever les liaisons d'aldéhyde sous forme de gaz, du dinitrophénylhydrazine et des petits tubes adsorbeurs XAD.

7. Procédé selon une des revendications précédentes, **caractérisé par le fait que** les procédés chimiques d'analyse des liaisons volatiles, d'origine microbienne incluent des procédés de chromatographie gazeuse et/ou de chromatographie liquide et/ou de spectroscopie UV et/ou de spectroscopie de masse.

8. Procédé selon une des revendications précédentes, **caractérisé par le fait que** l'analyse des métaux (lourds) inclut la spectroscopie d'émission d'atomes.

9. Procédé selon une des revendications précédentes, **caractérisé par le fait que** les procédés servant à l'analyse des liaisons volatiles d'origine microbienne et/ou les procédés d'analyse biochimique servant à l'analyse des composants de moisissure sous forme de particule incluent les procédés de biologie moléculaire et/ou immunobiologiques.

10. Procédé d'après une des revendications, **caractérisé par le fait que** les agents polluants liés à la poussière et/ou les particules polluantes sont collectées à l'aide d'un aspirateur, sachant qu'un sac neuf a été inséré dans l'aspirateur et/ou que la poussière est collectée à l'aide d'un embout avec une taille définie de grain, et qu'une analyse chimique et/ou biochimique vient s'y rajouter.

11. Procédé selon une des revendications précédentes, **caractérisé par le fait que** les composants de moisissure liés à la poussière et/ou présents sous forme de particules sont prélevés à l'aide d'un matériau porteur en tissu, en papier ou semblable à un film, et sont ensuite présentés à une analyse de biologie moléculaire et/ou immunobiologique.

12. Procédé selon une des revendications précédentes, **caractérisé par le fait que** la poussière collectée lors du prélèvement d'échantillons est vieille d'un à plusieurs, de préférence sept jours et sert à prouver la présence de liaisons organiques peu volatiles et/ou de liaisons organiques sous forme de particules et/ou de métaux (lourds) et/ou de moisissures.

13. Kit d'analyse pour exécuter le procédé d'après une des revendications 1-12 avec des matériaux d'adsorption et des matériaux porteurs pour le prélèvement d'échantillons afin de procéder ultérieurement à une analyse destinée à prouver la présence d'agents polluants (analyse d'agents polluants) dans les espaces intérieurs, sachant que
• le prélèvement d'échantillons sert tant à l'analyse de l'air quant à la présence d'agents polluants volatils qu'à celle d'attaques de moisissure, et que
• le kit d'analyse contient un ou divers agents d'adsorption liés dans des petits tubes d'adsorption pour la sorption de substances sous forme de gaz, ainsi que pour prouver les liaisons organiques peu volatiles, les liaisons organiques sous forme de particules et/ou de métaux (lourds).

14. Kit d'analyse des agents polluants selon la revendication 13, **caractérisé par le fait que** le kit peut contenir des appareils de prélèvement d'échantillons, notamment une pompe avec des raccords compatibles aux raccordements des petits tubes adsorbeurs.

15. Kit d'analyse dés agents polluants selon la revendication 13 et 14, **caractérisé par le fait que** le matériau porteur pour le prélèvement d'agents polluants liés à la poussière et sous forme de particules sont des sacs pour aspirateur ou des têtes de prélèvement d'échantillons destinés à être posés sur l'aspirateur avec une taille variable de grain et/ou des chiffons pour essuyer.

16. Kit d'analyse des agents polluants selon une des revendications 13 à 16, **caractérisé par le fait que** les agents d'adsorption et les matériaux porteurs sont inertes par rapport aux analytes.

17. Kit d'analyse des agents polluants selon une des revendications 13 à 16, **caractérisé par le fait qu'**au moins un des agents d'adsorption peut faire passer un ou plusieurs analytes dans un état chimique stable.

18. Kit d'analyse des agents polluants selon une des revendications 13 à 17, **caractérisé par le fait que** les petits tubes adsorbeurs sont remplis avec
• du charbon actif,
• du nitiophénylhydrazine,
• des zéolithes,
• du gel de silice,
• des polyuréthanes ou un agent d'adsorption semblable.

19. Kit d'analyse d'agents polluants selon une des revendications 13 à 18, **caractérisé par le fait que** les petits tubes adsorbeurs sont compatibles avec des appareils de prélèvement d'échantillons et des appareils d'analyse.

20. Kit d'analyse des agents polluants selon une des revendications 13 à 20, **caractérisé par le fait que** le kit d'analyse contient aussi des récipients et/ou des enveloppes de protection et/ou des enveloppes pour l'envoi selon les règles des échantillons et des agents d'adsorption.

21. Utilisation du kit d'analyse décrit selon une des revendications 13 à 20 pour l'analyse des agents polluants dans les espaces intérieurs selon le procédé d'une des revendications 1 à 13.
